# EUROPEAN PATENT APPLICATION

(11) **EP 2 394 674 A2**
(43) Date of publication of application: **14.12.2011**
(21) Application number: 11305578.4
(22) Date of filing: 12.05.2011
(51) Int. Cl.: A61L 27/50

(54) **Reactive Surgical Implants**

(30) Priority: 12.05.2010 US 778348
(71) Applicant: Tyco Healthcare Group LP, New Haven, CT 06511 (US); SOFRADIM PRODUCTION, 01600 Trévoux (FR)
(72) Inventor: SARGEANT, Timothy, Guilford, CT 06437 (US); STOPEK, Joshua, GUILFORD, CT 06437 (US); LADET, Sébastien, 69006, LYON (FR); GRAVAGNA, Philippe, 69540, IRIGNY (FR); ELACHCHABI, Amin, HAMDEN, CT 06518 (US)
(74) Representative: Soames, Candida Jane

(57) **Abstract**

The present disclosure relates to a sprayable surgical implant. The implant includes a first component including microparticulates and a second component including at least one cross-linking reagent. The at least one cross-linking reagent reacts with the microparticulates to form the surgical implant.

## Description

### BACKGROUND

The present disclosure relates to a surgical implant and more particularly to implants for soft tissue repair.

Many surgical implants, for example, surgical meshes, may be used in situ to provide support to soft tissue. Surgical meshes may be porous or non-porous. Those that are porous may allow for tissue in-growth following implantation and during the healing process.

Porous surgical implants may be preformed by methods such as freeze drying, salt leaching from foams, or forming the mesh from a textile. Other implants have been developed that may be formed in situ. These in situ forming implants may be deliverable during minimally invasive procedures and may be able to conform to complex tissue architecture. Such in situ forming implants may take the form of hydrogels. These hydrogels may lack the porous structure that allows for tissue in-growth, forming a seal rather than an integrated support.

It would be advantageous to formulate a surgical implant that is porous, conforms to tissue architecture, and is delivered in a minimally invasive manner.

### SUMMARY

The present disclosure provides surgical implants and methods for making same. In embodiments, a surgical implant of the present disclosure may include a first component including microparticulates having a size of from about 10 pm to about 500 pm, and a second component including at least one cross-linking reagent. In embodiments, the first component, the second component, or both, may be in solution.

In other embodiments, a surgical implant of the present disclosure may include a first component including surface modified protein microparticulates, and a second component including at least one cross-linking reagent, wherein the surface modified protein microparticulates have a size of from about 10 µm to about 500 µm.

Methods for forming implants are also provided herein. In embodiments, a method of the present disclosure may include forming a first solution including microparticulates having a size of from about 10 µm to about 500 µm, forming a second solution including at least one cross-linking reagent, introducing the first solution and the second solution onto tissue, and allowing the at least one cross-linking reagent to react with the microparticulates in situ, thereby forming a porous surgical implant.

### BRIEF DESCRIPTION OF THE DRAWINGS

Various embodiments of the present disclosure will be described herein below with reference to the following figures wherein:

FIGS. 1A-1D are illustrations depicting the use of a surgical implant of the disclosure for cartilage repair; and

FIGS. 2A-2D are illustrations depicting the use of a surgical implant of the disclosure for hernia repair.

### DETAILED DESCRIPTION

The present disclosure provides hydrogels which include at least two components: a first component including microparticulates and a second component including at least one cross-linking reagent. The microparticulate component and cross-linker react upon contact to form an open and porous matrix. In embodiments, this porous matrix may be formed in situ, thereby forming an implant.

As used herein, the term "microparticulate" or "microparticulates" may include any nano, meso, or micro-sized particles, having a diameter from about 10 µm to about 500 pm, in embodiments, from about 50 µm to about 250 µm. Microparticulates may be of any shape, including microrods, microfibers, microbeads, irregular shaped, spherical, non-spherical, combinations thereof, and the like. The structure of the microparticulates may be solid, semisolid, hollow, or any combinations of these.

The microparticulates may be formed from materials including: natural biodegradable polymers; lipids; synthetically modified natural polymers; synthetic degradable polymers; non-biodegradable polymers; and combinations of the foregoing.

Representative natural biodegradable polymers which may be used to form the microparticulates include polysaccharides, lipids, proteins, combinations thereof, and the like. Suitable polysaccharides include alginate, dextran, chitin, hyaluronic acid, cellulose, fucans, and glycosaminoglycans, as well as chemical derivatives thereof such as aminated dextran, aminated cellulose and aminated hyaluronic acid. Polysaccharides may also be modified, including substitutions and/or additions of chemical groups, for example, alkyl, alkylene, hydroxylations, oxidations, and other modifications within the purview of those skilled in the art. Suitable lipids include glycerides; cutin; lecithin; tocopherols, including alpha tocopherol, beta tocopherol and gamma tocopherol; vegetable oils such as olive oil, coconut oil, corn oil, cottonseed oil, palm oil, rapeseed oil, almond oil, cashew oil, hazelnut oil, macadamia oil, mongongo oil, pine nut oil, pistachio oil, walnut oil, bottle gourd oil, buffalo gourd oil, pumpkin seed oil, watermelon seed oil, acai oil, blackcurrant seed oil, borage seed oil, evening primrose oil, carob pod oil, amaranth oil, apricot oil, apple seed oil, argan oil, artichoke oil, avocado oil, babassu oil, ben oil, borneo tallow nut oil, cape chestnut oil, cocoa butter, algaroba oil, cocklebur oil, poppyseed oil, cohune oil, dika oil, false flax oil, flax seed oil, grape seed oil, hemp oil, kapok seed oil, lallemantia oil, marula oil, meadowfoam seed oil, mustard oil, nutmeg butter, nutmeg oil, okra seed oil (hibiscus seed oil), papaya seed oil, perilla seed oil, pequi oil, pine nut oil, poppyseed oil, prune kernel oil, quinoa oil, ramtil oil, rice bran oil, royle oil, rye oil, sacha inchi oil, tea oil (camellia oil), thistle oil, tomato seed oil, and wheat germ oil; combinations thereof, and the like. Suitable proteins include albumin, collagen, gelatin, casein, zein, silk, elastin, combinations thereof, and the like.

Synthetically modified natural polymers which may be utilized to form the microparticulates include cellulose derivatives, such as alkyl celluloses, hydroxyalkyl celluloses, cellulose ethers, cellulose esters, nitrocelluloses, chitosan, combinations thereof, and the like. Examples of suitable cellulose derivatives include aminated cellulose, methyl cellulose, ethyl cellulose, hydroxypropyl cellulose, hydroxypropyl methyl cellulose, hydroxybutyl methyl cellulose, cellulose acetate, cellulose propionate, cellulose acetate butyrate, cellulose acetate phthalate, carboxymethyl cellulose, cellulose triacetate, and cellulose sulfate sodium salt. These may be collectively referred to herein as "celluloses." Synthetically modified natural polymers also include recombinant synthetic proteins, recombinant synthetic peptides, and copolymers and combinations thereof.

Representative synthetic degradable polymers which may be utilized to form the microparticulates include polyhydroxy acids prepared from lactone monomers such as glycolide, lactide, caprolactone, _{E}-caprolactone, valerolactone, 6-valerolactone, combinations thereof, and the like; carbonates such as trimethylene carbonate, combinations thereof, tetramethylene carbonate, combinations thereof, and the like; dioxanones such as 1,4-dioxanone and p-dioxanone; 1,dioxepanones such as 1,4-dioxepan-2-one and 1,5-dioxepan-2-one; combinations thereof, and the like. Polymers formed therefrom include: polylactides; poly(lactic acid); polyglycolides; poly(glycolic acid); poly(trimethylene carbonate); poly(dioxanone); poly(hydroxybutyric acid); poly(hydroxyvaleric acid); poly(lactide-co-(E-caprolactone)); poly(glycolide-co-(E-caprolactone)); polycarbonates; combinations thereof, and the like.

Other polymers which may be utilized to form the microparticulates include poly(pseudo amino acids); poly(amino acids); poly(hydroxyalkanoate)s; polyalkylene oxalates; polyoxaesters; polyanhydrides; polyortho esters; and copolymers, block copolymers, homopolymers, blends, combinations thereof, and the like.

Rapidly bioerodible polymers, such as poly(lactide-co-glycolide)s, polyanhydrides and polyorthoesters, which have carboxylic groups exposed on their surface, especially as the smooth surface of the polymer erodes in vivo, may also be used to form the microparticulates.

Some non-limiting examples of suitable non-bioabsorbable materials from which the microparticulates may be made include: polyolefins, such as polyethylene and polypropylene, including atactic, isotactic, syndiotactic, and combinations thereof; polyethylene glycols; polyethylene oxides; ultra high molecular weight polyethylene; copolymers of polyethylene and polypropylene; polyisobutylene and ethylene-alpha olefin copolymers; fluorinated polyolefins, such as fluoroethylenes, fluoropropylenes, fluoroPEGs, and polytetrafluoroethylene; polyamides, such as nylon and polycaprolactam; polyamines; polyimines; polyesters, such as polyethylene terephthalate and polybutylene terephthalate; aliphatic polyesters; polyethers; polyether-esters, such as polybutester; polytetramethylene ether glycol; 1,4-butanediol; polyurethanes; acrylic polymers and copolymers; methacrylic polymers; biocompatible vinyl halide polymers and copolymers, such as polyvinyl chloride; polyvinyl alcohols; polyvinyl ethers, such as polyvinyl methyl ether; polyvinylidene halides, such as polyvinylidene fluoride and polyvinylidene chloride; polyacrylonitrile; polyaryletherketones; polyvinyl ketones; polyvinyl aromatics, such as polystyrene; polyvinyl esters, such as polyvinyl acetate; vinyl polymers, including copolymers of vinyl monomers with each other and olefins, such as ethylene-methyl methacrylate copolymers, acrylonitrile-styrene copolymers, ABS resins, and ethylene-vinyl acetate copolymers; phosphorocholine based vinyl polymers; polyvinyl pyrrolidone; 2-hydroxyethyl methacrylic acid; alkyd resins; polycarbonates; polyoxymethylenes; polyphosphazine; polyimides; epoxy resins; aramids, rayon; rayon-triacetate; spandex; silicones; polyethylene oxide/polypropylene oxide copolymers, including those commercially available as PLURONICS; combinations thereof; and the like.

The microparticulates, in embodiments microspheres, may be prepared by any method within the purview of those skilled in the art, including, but not limited to, spray drying, emulsion, double emulsion, extrusion, ultrasonic generation, combinations thereof, and the like. The microparticulates may be of a single size or various sizes, with either a broad or narrow size distribution.

The microparticulates may be reactive. This reactivity may be derived by functionalizing the microparticulates. The microparticulates may be functionalized with any group capable of reacting with a cross-linking reagent. Reactive groups that may be utilized include, for example, NH₂, COOH, S0₃, COH, aldehydes, sulfones, vinylsulfones, isocyanates, acid anhydrides, combinations thereof, and the like. Other reactive groups that may be utilized include, for example,
- CO₂N(COCH₂)₂, -CO₂N(COCH₂)₂, -CO₂H, -CHO, -CHOCH₂, -N=C=O,
- SO₂CH=CH₂, -N(COCH)₂, -S-S-(C₅H₄N), combinations thereof, and the like.

In embodiments, the microparticulates may be in a solution, thereby forming what may be referred to, in embodiments, as a first solution. Any biocompatible solvent may be used to form this first solution. In embodiments, the first solution may be formed by diluting the microparticulates in a buffer solution. The buffer solution may be, for example, phosphate buffered saline (PBS), Hanks buffered salt solution, water, combinations thereof, and the like. The concentration of microparticulates in solution may be from about 0.5% to about 50%, in embodiments from about 1 % to about 25%.

In embodiments, more than one type of microparticulate may be in a buffer solution, such as a mixture of collagen and elastin microparticulates.

The composition of the present disclosure also includes at least one cross-linking reagent as a second component. Cross-linking reagents may result in cross-linking of the microparticulate component by any method of cross-linking within the purview of those skilled in the art. Cross-linking reagents may include those based upon reactive NHS chemistry, UV-based cross-linkers, sugar-based cross-linkers, silicone coupling agents, combinations thereof, and the like.

Reactive components that may be used as the cross-linking reagent include, but are not limited to, reactive silicones, isocyanates, N-hydroxy succinimides ("NHS"), cyanoacrylates, aldehydes (e.g., formaldehydes, glutaraldehydes, glyceraldehydes, and dialdehydes), genipin, and other compounds possessing chemistries having some affinity for the microparticulates, tissue, or both. As used herein, succinimides also include sulfosuccinimides, succinimide esters and sulfosuccinimide esters, including N-hydroxysuccinimide ("NHS"), N-hydroxysulfosuccinimide ("SNHS"), N-hydroxyethoxylated succinimide ("ENHS"), N-hydroxysuccinimide acrylate, succinimidyl glutarate, n-hydroxysuccinimide hydroxybutyrate, combinations thereof, and the like. In embodiments, the reactive component may be any reactive component as described in U.S. Patent Nos. 6,566,406, 6,818,018, 7,009,034, 7,025,990, 7,211,651, 7,332,566, the entire disclosures of each of which are incorporated by reference herein. The reactive component may be combined with any other component utilizing any method within the purview of those skilled in the art, including those disclosed in U.S. Patent Nos. 6,566,406, 6,818,018, 7,009,034, 7,025,990, 7,211,651, and/or 7,332,566, the entire disclosures of each of which are incorporated by reference herein. Cross-linking reagents utilized in accordance with the present disclosure may also include any natural or synthetic crosslinkers including, but not limited to, aldehydes such as those listed above; diimides; diisocyanates; cyanamide; carbodiimides; dimethyl adipimidate; starches; combinations thereof, and the like.

Suitable cross-linking reagents may include, in embodiments, amine reactive groups, for example, isocyanate groups, isothiocyanates, diimidazoles, imidoesters, hydroxysuccinimide esters, and aldehydes. Examples include, but are not limited to, aromatic diisocyanates such as 2,4-toluene diisocyanate, 2,6-toluene diisocyanate, 2,2'-diphenylmethane diisocyanate, 2,4'-diphenylmethane diisocyanate, 4,4'-diphenylmethane diisocyanate, diphenyldimethylmethane diisocyanate, dibenzyl diisocyanate, naphthylene diisocyanate, phenylene diisocyanate, xylylene diisocyanate, 4,4'-oxybis(phenylisocyanate), tetramethylxylylene diisocyanate, tolylenediisocyanate, benzoyl isocyanates, and m-tetramethylxylylenediisocyanate; aliphatic diisocyanates such as tetramethylene diisocyanate, hexamethylene diisocyanate (HMDI), dimethyl diisocyanate, lysine diisocyanate, 2-methylpentane-1,5-diisocyanate, 3-methylpentane-1,5-diisocyanate, 2,2,4-trimethylhexamethylene diisocyanate, and butane diisocyanate; and alicyclic diisocyanates such as isophorone diisocyanate, cyclohexane diisocyanate, hydrogenated xylylene diisocyanate, hydrogenated diphenylmethane diisocyanate, hydrogenated trimethylxylylene diisocyanate, 2,4,6-trimethyl 1,3-phenylene diisocyanate, or those commercially available as DESMODURS ^{Ⓡ} from Bayer Material Science. Other suitable isocyanates include, for example, para-phenylene diisocyanate, p-phenylacetylisocyanate, m-phenylacetylisocyanate, m-phenoxyacetylisocyanate, p-phenoxyacetylisocyanate, and m-hydrocinnamylisocyanate.

In embodiments, the cross-linking reagent may include a macromer endcapped with a diisocyanate. Suitable macromers include polyethers, polyesters, combinations thereof, and the like. Methods of endcapping a polyether, polyester, or poly(ether-ester) macromer with a diisocyanate are within the purview of those skilled in the art. For example, the polyether, polyester, or poly(ether-ester) macromer may be combined with a suitable diisocyanate at a molar ratio of polyether, polyester or poly(ether-ester) macromer to diisocyanate of from about 1:2 to about 1:6, in embodiments from about 1:3 to about 1:5, in other embodiments about 1:4, and heated to a suitable temperature of from about 55° C to about 75° C, in embodiments from about 60° C to about 70° C, in other embodiments about 65° C. It may be desirable to agitate the components utilizing means within the purview of those skilled in the art, including stirring, mixing, blending, sonication, combinations thereof, and the like.

In some embodiments, the endcapping reaction may occur under an inert atmosphere, for example, under nitrogen gas. Catalysts, including alkoxides, stannous octoate, dibutyltin dilaurate, 1,4-diazabicyclo[2.2.2]octane (DABCO), combinations thereof, and the like, may be utilized in some embodiments to increase the rate of the endcapping reaction.

It may be desirable, in embodiments, to utilize an excess of diisocyanate in carrying out the reaction. The use of an excess of diisocyanate may suppress the polymerization reaction, thereby permitting one to tailor the resulting molecular weight of the resulting isocyanate functionalized second component. In some embodiments the resulting diisocyanate-functional compound may then be obtained by hot extraction with petroleum ether.

In some embodiments, suitable macromers which may be utilized as the cross-linking reagents include those of the following formula: 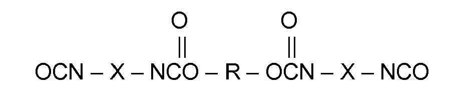 wherein R is a polyether, a polyester or a polyether-ester as described above; and X is an aromatic, aliphatic, or alicyclic group as described above.

In other embodiments, a diisocyanate compound which may be used as the cross-linking reagent may be of the following formula:

OCN - X - HNCOO - (R - A)ₙ -R - OOCNH - X - NCO

wherein X is an aliphatic or aromatic group; A is a degradable group, in embodiments derived from an aliphatic diacid; R can be the same or different at each occurrence and is a group derived from a dihydroxy compound; and n is 1 to 10. In some embodiments, X may be derived from toluene, hexamethylene, tetramethylene, lysine, ethylated lysine isophorone, xylene, diphenylmethane, diphenyldimethylmethane, dibenzyl diisocyanate, oxybis(phenylisocyanate), tetramethylxylylene, or optionally combinations thereof.

The cross-linking reagents may be at least bi-functional in nature, i.e., forming a linear molecule. In embodiments, the cross-linking reagent may be multifunctional, forming a macromer molecule, such as, for example, a multi-arm polyethylene glycol (PEG).

In embodiments, the cross-linking reagent can be a multi-arm polyethylene glycol with a succinimide reactive group capable of reacting with primary amines present on microparticulates.

The cross-linking reagent, like the microparticulates, may be in a solution. Any biocompatible solvent may be used to form such a solution. In embodiments, the solution may be aqueous. The concentration of cross-linking reagent in the solution may be, for example, about 5% to about 95% by weight. Alternatively, the cross-linking reagent may be a liquid that does not require dilution in solution such as, for example, a low molecular weight polyethylene glycol (having a molecular weight, in embodiments, of about 2000) functionalized with NHS reactive groups.

Biological cross-linking systems may also be utilized, including: antibody/antigen; biotin/avidin; complimentary peptide binding sequences; nucleotide base pairing and cross-linking; hybrid "click" chemistry methods; chemical cross-linking, such as Huisgen cycloaddition, Diels-Alder reactions, thiol-alkene reactions, and maleimide-thiol reactions; lock and key protein binding chemistry; self-assembling peptides, combinations thereof, and the like.

In embodiments, the cross-linking reagent may be a polymer having at least one reactive group known to have click reactivity, capable of reacting via click chemistry. Click chemistry refers to a collection of reactive groups having a high chemical potential energy capable of producing highly selective, high yield reactions. Examples of click chemistry reactions which may be utilized in accordance with the present disclosure include those disclosed in U.S. Patent Application Serial No. 12/368415, the entire disclosure of which is incorporated by reference herein.

In embodiments, the microparticulate component contains biotin and/or avidin. For example, in some embodiments, the cross-linking component may contain avidin and the microparticulate component may contain biotin. In other embodiments, the cross-linking component may contain biotin and the microparticulate component may contain avidin.

In embodiments, the microparticulates and/or the cross-linking reagent may react in situ to form a mesh, a scaffold, a plug, a void filler, similar structures, and the like.

The microparticulates and/or the cross-linking reagent may also function as a delivery mechanism for bioactive agents or therapeutics. A bioactive agent as used herein is used in the broadest sense and includes any substance or mixture of substances that have clinical use. Consequently, bioactive agents may or may not have pharmacological activity per se, e.g., a dye. Alternatively a bioactive agent could be any agent that provides a therapeutic or prophylactic effect; a compound that affects or participates in tissue growth, cell growth, and/or cell differentiation; an anti-adhesive compound; a compound that may be able to invoke a biological action such as an immune response; or could play any other role in one or more biological processes. A variety of bioactive agents may be incorporated into either or both solutions forming the surgical implant.

Examples of classes of bioactive agents, which may be utilized in accordance with the present disclosure include, for example, anti-adhesives, antimicrobials, analgesics, antipyretics, anesthetics, antiepileptics, antihistamines, anti-inflammatories, cardiovascular drugs, diagnostic agents, sympathomimetics, cholinomimetics, antimuscarinics, antispasmodics, hormones, growth factors, muscle relaxants, adrenergic neuron blockers, antineoplastics, immunogenic agents, immunosuppressants, gastrointestinal drugs, diuretics, steroids, lipids, lipopolysaccharides, polysaccharides, platelet activating drugs, clotting factors and enzymes. It is also intended that combinations of bioactive agents may be used.

Other optional bioactive agents include: local anesthetics; non-steroidal antifertility agents; parasympathomimetic agents; psychotherapeutic agents; tranquilizers; decongestants; sedative hypnotics; steroids; sulfonamides; sympathomimetic agents; vaccines; vitamins; antimalarials; anti-migraine agents; anti-parkinson agents such as L-dopa; anti-spasmodics; anticholinergic agents (e.g., oxybutynin); antitussives; bronchodilators; cardiovascular agents, such as coronary vasodilators and nitroglycerin; alkaloids; analgesics; narcotics such as codeine, dihydrocodeinone, meperidine, morphine and the like; non-narcotics, such as salicylates, aspirin, acetaminophen, d-propoxyphene and the like; opioid receptor antagonists, such as naltrexone and naloxone; anti-cancer agents; anti-convulsants; anti-emetics; antihistamines; anti-inflammatory agents, such as hormonal agents, hydrocortisone, prednisolone, prednisone, non-hormonal agents, allopurinol, indomethacin, phenylbutazone and the like; prostaglandins and cytotoxic drugs; chemotherapeutics; estrogens; antibacterials; antibiotics; anti-fungals; anti-virals; anticoagulants; anticonvulsants; antidepressants; antihistamines; and immunological agents.

Other examples of suitable bioactive agents include, for example, viruses and cells; peptides, polypeptides and proteins, as well as analogs, muteins, and active fragments thereof; immunoglobulins; antibodies; cytokines (e.g., lymphokines, monokines, chemokines); blood clotting factors; hemopoietic factors; interleukins (IL-2, IL-3, IL-4, IL-6); interferons (β-iFN, α-IFN and y-IFN); erythropoietin; nucleases; tumor necrosis factor; colony stimulating factors (e.g., GCSF, GM-CSF, MCSF); insulin; anti-tumor agents and tumor suppressors; blood proteins such as fibrin, thrombin, fibrinogen, synthetic thrombin, synthetic fibrin, synthetic fibrinogen; gonadotropins (e.g., FSH, LH, CG, etc.); hormones and hormone analogs (e.g., growth hormone); vaccines (e.g., tumoral, bacterial and viral antigens); somatostatin; antigens; blood coagulation factors; growth factors (e.g., nerve growth factor, insulin-like growth factor); bone morphogenic proteins; TGF-B; protein inhibitors; protein antagonists; protein agonists; nucleic acids, such as antisense molecules, DNA, RNA, RNAi; oligonucleotides; polynucleotides; and ribozymes.

In embodiments, the bioactive agent may be small molecule drugs such as anesthetics, angiogenics, anti-spasmodics, non-steroidal anti-inflammatories, steroids, combinations of these, and the like. In embodiments, the bioactive agent may be a large molecule drug such as a protein or growth factor. In other embodiments, the bioactive agent is a biologic or cell specific ligand capable of attracting or recruiting specific cell types, such as smooth muscle cells, stem cells, immune cells, and the like.

In embodiments, the surface of the microparticulates may be modified with bioactive peptides, including fibronectin, laminin, thrombospondin, combinations thereof, and the like. In embodiments, the microparticulates may contain within, or be surface modified to contain, drugs or bioactive agents for pain; oncology; angiogenesis; wound healing; spasms; clotting; infection; combinations thereof, and the like. In embodiments, the surface modified microparticulates may be an aminated or thiolated polymer, polyethylene terephthalate (PET). In embodiments, the microparticulates may encapsulate cellular therapeutics including: stem cells; chrondrocytes; immunocompetent cells; neural cells; glial cells; vascular cells; combinations thereof, and the like. The microparticulates may also contain a local anesthetic, such as bupivacaine.

The bioactive agent may be contained within the microparticulates through bulk or post loading. In embodiments, the microparticulates may be surface modified to contain the bioactive agent. In embodiments, the bioactive may be incorporated into the buffer or other solution used to dilute either the first or second solution.

The amounts of microparticulates combined with a cross-linking reagent to form a hydrogel of the present disclosure may be adjusted depending on the intended use of the hydrogel. In embodiments, the microparticulates may be present in an amount of from about 75% by weight to about 99% by weight of a hydrogel of the present disclosure, in embodiments from about 90% by weight to about 98% by weight of a hydrogel of the present disclosure, and the cross-linking reagent may be present in an amount of from about 1 % by weight to about 25% by weight of a hydrogel of the present disclosure, in embodiments from about 2% by weight to about 10% by weight of a hydrogel of the present disclosure.

Once formed, the hydrogel will form a hydrogel system as it takes in water. A hydrogel system may include up to about 99% water, in embodiments from about 90% to about 99% water, in embodiments from about 93% to about 97% water.

Delivery of the first and second components may be during open or minimally invasive surgery. The resulting hydrogels of the present disclosure may thus form a surgical implant that is porous and conforms to tissue architecture. The microparticulate component and cross-linker may be introduced in vivo, in embodiments by minimally invasive procedures, such as endoscopic, laparoscopic, arthroscopic, endoluminal and/or transluminal placement of the two components. In embodiments, the components may be in solution and applied with an applicator that includes at least two reservoirs, one for each solution. The reservoirs may be, for example, syringes, single or multi-lumen tubing, catheters, flexible pouches or bags, or other conduits which may push or extrude their contents into a mixing chamber and/or expel the components through a mixing tip. The mixing tip may be fitted with a static molding or spray attachment at the distal end of the mixer to assist with preferred delivery. Gas, fluids or other forms of propellant may be used to deliver the solutions.

In embodiments, the hydrogels may be delivered in a minimally invasive manner, such as laparoscopically. Laparoscopic surgical procedures are minimally invasive procedures, which are carried out within the body cavity through use of access ports in conjuncture with elongated surgical devices. An initial opening in the body tissue enables passage of the endoscopic or laparoscopic device to the interior of the body. Openings include natural passageways of the body or openings which are created by a tissue piercing device such as a trocar. During laparoscopic procedures, narrow punctures or incisions are made minimizing trauma to the body cavity and reducing patient recovery time.

Upon contact the reactive groups located on the microparticulates and the cross-linking reagent may react to form a porous surgical implant or matrix. The cross-linking reagent, microparticulates, or both, may also react with the tissue in situ. The microparticulates may add weight and volume to the surgical implant thereby providing structural support to the damaged area. As the microparticulates cross-link with the cross-linking reagent, the spaces between the microparticulates provide a porous area for tissue in-growth. This porous area also provides a measure of flexibility to the implant allowing it to flex with the surrounding tissue. Pores in the resulting implant may have a diameter of from about 1 µm to about 100 µm, in embodiments from about 5 pm to about 50 µm.

The rate of cross-linking of the microparticulates with the cross-linking reagent may be controlled by various means, for example, pH, ultraviolet light (UV), visible light, temperature, combinations thereof, and the like. The intensity of some of the above means, for example, any visible light or UV light, may also be used to control the rate of cross-linking. By controlling the rate of cross-linking, the thickness of the implant may be controlled. Depending on the cross-linking chemistry and density, the resulting matrix may be permanent or degradable, rigid or elastomeric, swellable, stable, or contractable.

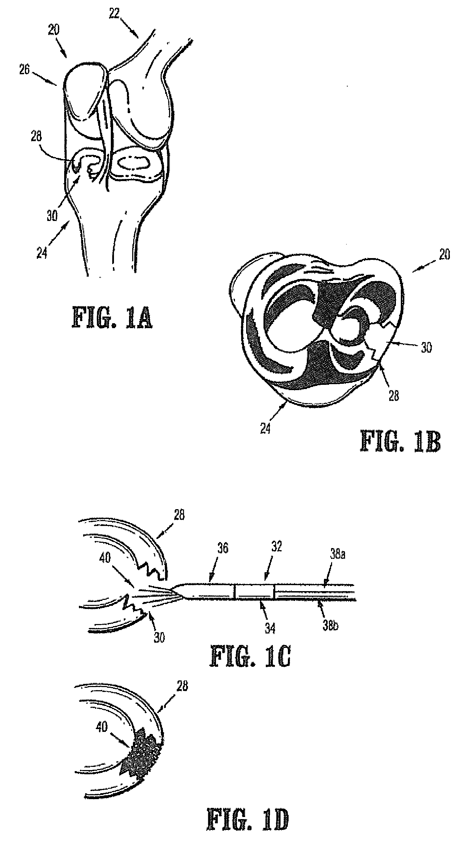

Referring now in specific detail to the figures, in which like numbers identify similar or identical elements, FIG. 1A is a perspective view of a knee 20, which includes femur 22, tibia 24, patella 26, and cartilage 28. The cartilage 28 includes a tear 30. FIG. 1B is a top view of a tibia 24 and cartilage 28. The tear 30 in the cartilage 28 is clearly visible. Repair of a tear 30 in cartilage 28 is illustrated in FIG. 1C utilizing a multi-lumen tubing 32 having a mixing chamber 34 and a spray tip 36. The first and second solutions 38a/38b, containing microparticulates and a cross-linking reagent, respectively, may be separated using the multi-lumen tubing 32 and mixed in the mixing chamber 34 prior to spraying into the tear 30 in the cartilage 28, whereby they mix to form hydrogel composition 40. FIG. 1D illustrates the porous surgical implant 40 formed in the tear 30 of the cartilage 28.

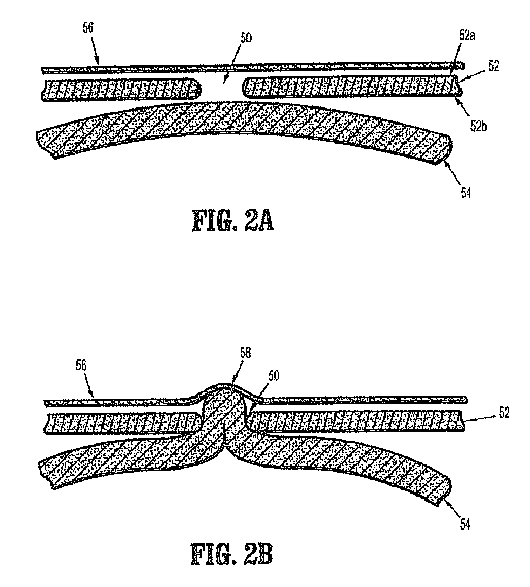

Referring now to FIGS. 2A-2D, an alternate method of using a surgical implant of the present disclosure in performing a surgical repair procedure is shown and described. With reference to FIG. 2A, a hernia may involve a tear 50, in the abdominal wall 52. Abdominal wall 52 is defined by an external side 52a and peritoneum 52b. A surface tissue 56, which covers the external side 52a of abdominal wall 52, may or may not be immediately affected by this tear 50. An internal organ 54 located below the peritoneum 52b of the abdominal wall 52 may not protrude until some form of exertion or use of the muscle located at the abdominal wall 52 forces the internal organ 54 into the tear 50. Depending on the size and location of the tear 50, exertion may not be needed to cause the organ 54 to protrude. As shown in FIG. 2B, a hernia occurs when internal organ 54 protrudes into the tear 50 of abdominal wall 52. Oftentimes the protrusion creates a bulge 58 in the surface tissue 56.

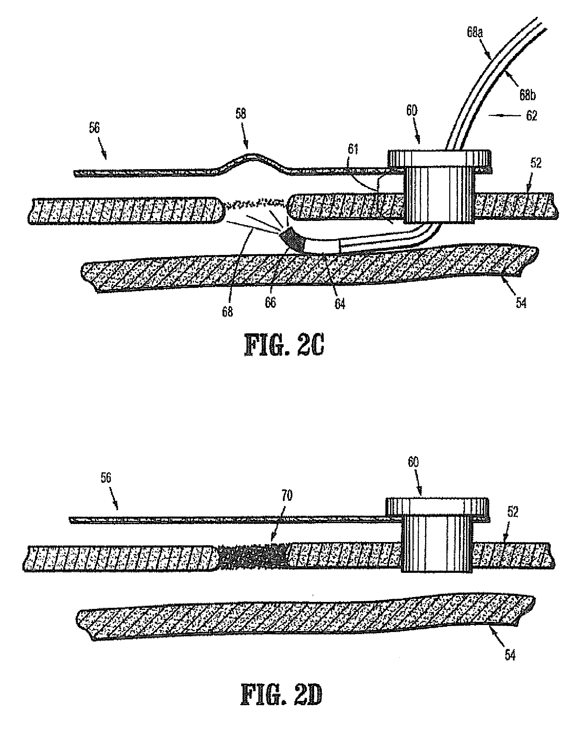

In order to correct the defect, as depicted in FIG. 2C, an incision 61 is made through the abdominal wall 52 in close proximity to tear 50 and a multi-lumen syringe or other device 62 is inserted using a trocar 60 or similar laparoscopic device. The multi-lumen syringe 62 may include a mixer 64 and a spray tip 66 to mix and spray the first and second solutions 68a/68b, containing microparticulates and a cross-linking reagent, respectively, into the tear 50 as mixture 68. As shown in FIG. 2D, the solutions 68a/68b cross-link to form a porous surgical implant 70.

As illustrated above, the solutions may be sprayed in situ to form a surgical implant. The surgical implant may both provide mechanical support and encourage tissue in-growth. The surgical implant may be used in repair of, for example: ventral hernias; inguinal hernia repairs; cartilage; bone; dermal filler; skin flaps; trocar incision sites; anastomotic; neural defects; and the like.

The following Examples are being submitted to illustrate embodiments of the present disclosure. These Examples are intended to be illustrative only and are not intended to limit the scope of the present disclosure. Also, parts and percentages are by weight unless otherwise indicated.

### EXAMPLES

### EXAMPLE 1

An emulsion based method may be used for forming collagen or serum albumin microspheres for a first solution. The microspheres are suspended in a buffer at a concentration of from about 20 to about 200 mg/ml, and the suspended microspheres are loaded into a first syringe.

A second solution may be prepared from a multi-arm reactive PEG NHS ester cross-linking reagent in a buffer at a concentration of from about 50 to about 100 mg/ml. The second solution is loaded into a second syringe.

The syringes are loaded into an applicator equipped with a static mixer and a spray tip. The primary amines on the surface of the protein microspheres cross-link with the PEG NHS ester upon contact and form an insoluble hydrogel network with an open pore structure. The rate of cross-linking is controlled by the pH of the solution.

### EXAMPLE 2

The process of Example 1 is followed, except the second solution is formed from a degradable functionalized isocyanate cross-linking reagent in a buffer at a concentration of from about 50 to about 100 mg/ml.

### EXAMPLE 3

The process of Example 1 is followed, except collagen and elastin microspheres are utilized in a first solution. The microspheres are formed using an emulsion based method.

### EXAMPLE 4

Example 1 is followed, except microspheres are formed from polyethylene terephthalate (PET) using an emulsion based method. The microspheres are surface modified to include an amine and/or thiol group. The microspheres are suspended in a buffer at a concentration of from about 20 to about 200 mg/ml, and loaded into a first syringe.

### EXAMPLE 5

Microparticulates may be formed as per Example 1 above and surface modified to contain biotin and/or avidin. The microparticulates are suspended in a buffer at a concentration of from about 20 to about 200 mg/ml, and loaded into a first syringe.

The second solution has an avidin cross-linking reagent where the microparticulate component includes biotin, or a biotin cross-linking agent where the microparticulate component includes avidin.

The biotin and avidin will cross-link upon mixing, thereby forming an implant of the present disclosure.

While several embodiments of the disclosure have been described, it is not intended that the disclosure be limited thereto, as it is intended that the disclosure be as broad in scope as the art will allow and that the specification be read likewise. Therefore, the above description should not be construed as limiting, but merely as exemplifications of embodiments of the present disclosure. Various modifications and variations of the components used to form the surgical implant, as well as methods of delivering the components will be apparent to those skilled in the art from the foregoing detailed description. Such modifications and variations are intended to come within the scope and spirit of the claims appended hereto.

The invention may be described by reference to the following numbered paragraphs:
1. A surgical implant comprising a first component comprising microparticulates having a size of from about 10 µm to about 500 pm; and a second component comprising at least one cross-linking reagent.
2. The surgical implant of paragraph 1, wherein the second component comprises microparticulates surface modified with the at least one cross-linking reagent.
3. The surgical implant of paragraph 1, wherein the microparticulates comprise a polymer selected from the group consisting of polyolefins, polyesters, polyhydroxy acids, polysaccharides, lipids, polyamides, polyamines, vinyl polymers, and combinations thereof.
4. The surgical implant of paragraph 3, wherein the microparticulates comprise a polyhydroxy acid.
5. The surgical implant of paragraph 1, wherein the first component, the second component, or both, react utilizing a mechanism selected from the group consisting of biotin/avidin binding, antibody/antigen binding, peptide binding sequences, nucleotide base pairing, self-assembling peptides, lock and key protein binding, click chemistry, and combinations thereof.
6. The surgical implant of paragraph 1, wherein the first component and second component are cross-linked using a mechanism selected from the group consisting of UV-based systems, sugar-based systems, and combinations thereof.
7. The surgical implant of paragraph 1, wherein the first component, the second component, or both, further comprise at least one reactive group selected from the group consisting of N-hydroxy succinimides, reactive silicones, acrylates, aldehydes, isocyanates, and combinations thereof.
8. The surgical implant of paragraph 1, wherein the first component, the second component, or both, possess a reactive group comprising an amine.
9. The surgical implant of paragraph 1, further comprising a bioactive agent.
10. The surgical implant of paragraph 9, wherein the bioactive agent is selected from the group consisting of anesthetics, angiogenics, anti-spasmodics, anti-inflammatories, analgesics, antibiotics, and combinations thereof.
11. The surgical implant of paragraph 1, wherein the surgical implant comprises an in situ forming mesh.
12. The surgical implant of paragraph 1, wherein the surgical implant comprises an in situ forming scaffold.
13. The surgical implant of paragraph 1, wherein the first component, the second component, or both, possess a reactive group comprising a succinimide ester.
14. The surgical implant of paragraph 1, wherein the first component, the second component, or both, are in solution.
15. The surgical implant of paragraph 14, wherein both the first component and the second component are in solution, and wherein the concentration of the microparticulate in solution is from about 0.5% to about 50% by weight, and the concentration of the at least one cross-linking reagent in solution is from about 5% to about 95% by weight.
16. The surgical implant of paragraph 1, wherein the microparticulate comprises a shape selected from the group consisting of microspheres, microrods, microfibers, and combinations thereof.
17. A surgical implant comprising a first component comprising surface modified protein microparticulates; and a second component comprising at least one cross-linking reagent, wherein the surface modified protein microparticulates have a size of from about 10 pm to about 500 µm.
18. The surgical implant of paragraph 17, wherein the second component comprises microparticulates surface modified with the at least one cross-linking reagent.
19. The surgical implant of paragraph 17, wherein the surface modified protein microparticulates are selected from the group consisting of albumin, gelatin, casein, collagen, elastin, and combinations thereof.
20. The surgical implant of paragraph 17, wherein the surface modified protein microparticulates comprise collagen.
21. The surgical implant of paragraph 17, wherein the cross-linking reagent comprises a polyethylene glycol possessing a reactive group selected from the group consisting of N-hydroxysuccinimide, N-hydroxysulfosuccinimide, N-hydroxyethoxylated succinimide, N-hydroxysuccinimide acrylate, succinimidyl glutarate, n-hydroxysuccinimide hydroxybutyrate, and combinations thereof.
22. The surgical implant of paragraph 17, wherein the surface modified protein microparticulates comprise a bioactive agent.
23. The surgical implant of paragraph 22, wherein the bioactive agent comprises a peptide selected from the group consisting of fibronectin, laminin, thrombospondin, and combinations thereof.
24. The surgical implant of paragraph 17, wherein the surgical implant comprises an in situ forming mesh.
25. The surgical implant of paragraph 17, wherein the surgical implant comprises an in situ forming scaffold.
26. The surgical implant of paragraph 17, wherein the first component, the second component, or both, possess a reactive group comprising an amine.
27. The surgical implant of paragraph 17, wherein the first component, the second component, or both, possess a reactive group comprising a succinimide ester.
28. A method comprising forming a first solution comprising microparticulates having a size of from about 10 pm to about 500 pm; forming a second solution comprising at least one cross-linking reagent; introducing the first solution and the second solution onto tissue; and allowing the at least one cross-linking reagent to react with the microparticulates in situ thereby forming a porous surgical implant.
29. The method according to paragraph 28, wherein forming a first solution further comprises encapsulating a bioactive agent within the microparticulates.
30. The method according to paragraph 29, wherein the bioactive agent is selected from the group consisting of stem cells, chrondrocytes, immunocompetent cells, neural cells, glial cells, adipocytes, cardiac cells, muscle cells, endothelial cells, osteoblasts, vascular cells, and combinations thereof.
31. The method according to paragraph 29, wherein the bioactive agent comprises a local anesthetic.
32. The method according to paragraph 29, wherein the bioactive agent comprises a local analgesic.
33. The method according to paragraph 28, wherein forming a first solution further comprises modifying a surface of the microparticulates with a bioactive agent.
34. The method according to paragraph 28, wherein introducing the first solution and second solution onto tissue further comprises spraying onto tissue.
35. The method according to paragraph 29, wherein the bioactive agent is selected from the group consisting of a growth factor, peptide, DNA, siRNA, proteins, and combinations thereof.

## Claims

1. A surgical implant comprising:
a first component comprising microparticulates having a size of from about 10 pm to about 500 pm; and
a second component comprising at least one cross-linking reagent.

2. The surgical implant of claim 1, wherein the second component comprises microparticulates surface modified with the at least one cross-linking reagent, preferably wherein the microparticulates comprise a polymer selected from the group consisting of polyolefins, polyesters, polyhydroxy acids, polysaccharides, lipids, polyamides, polyamines, vinyl polymers, and combinations thereof; and/or wherein the microparticulates comprise a polyhydroxy acid.

3. The surgical implant of claim 1 or claim 2, wherein the first component, the second component, or both, react utilizing a mechanism selected from the group consisting of biotin/avidin binding, antibody/antigen binding, peptide binding sequences, nucleotide base pairing, self-assembling peptides, lock and key protein binding, click chemistry, and combinations thereof.

4. The surgical implant of any preceding claim, wherein the first component and second component are cross-linked using a mechanism selected from the group consisting of UV-based systems, sugar-based systems, and combinations thereof.

5. The surgical implant of any preceding claim, wherein the first component, the second component, or both, further comprise at least one reactive group selected from the group consisting of N-hydroxy succinimides, reactive silicones, acrylates, aldehydes, isocyanates, and combinations thereof.

6. The surgical implant of any preceding claim, wherein the first component, the second component, or both, possess a reactive group comprising an amine.

7. The surgical implant of any preceding claim, further comprising a bioactive agent, preferably wherein the bioactive agent is selected from the group consisting of anesthetics, angiogenics, anti-spasmodics, anti-inflammatories, analgesics, antibiotics, and combinations thereof.

8. The surgical implant of any preceding claim, wherein the surgical implant comprises an in situ forming mesh; and/or wherein the surgical implant comprises an in situ forming scaffold.

9. The surgical implant of claim 5, wherein the first component, the second component, or both, possess a reactive group comprising a succinimide ester.

10. The surgical implant of any preceding claim, wherein the first component, the second component, or both, are in solution, preferably wherein the concentration of the microparticulate in solution is from about 0.5% to about 50% by weight, and the concentration of the at least one cross-linking reagent in solution is from about 5% to about 95% by weight.

11. The surgical implant of any preceding claim, wherein the microparticulate comprises a shape selected from the group consisting of microspheres, microrods, microfibers, and combinations thereof.

12. A surgical implant comprising:
a first component comprising surface modified protein microparticulates; and
a second component comprising at least one cross-linking reagent,
wherein the surface modified protein microparticulates have a size of from about 10 µm to about 500 µm.

13. The surgical implant of claim 12, wherein the second component comprises microparticulates surface modified with the at least one cross-linking reagent.

14. The surgical implant of claim 12 or claim 13, wherein the surface modified protein microparticulates are selected from the group consisting of albumin, gelatin, casein, collagen, elastin, and combinations thereof, preferably wherein the surface modified protein microparticulates comprise collagen.

15. The surgical implant of claim 14, wherein the cross-linking reagent comprises a polyethylene glycol possessing a reactive group selected from the group consisting of N-hydroxysuccinimide, N-hydroxysulfosuccinimide, N-hydroxyethoxylated succinimide, N-hydroxysuccinimide acrylate, succinimidyl glutarate, n-hydroxysuccinimide hydroxybutyrate, and combinations thereof.

16. The surgical implant of any of claims 12 to 15, wherein the surface modified protein microparticulates comprise a bioactive agent, preferably wherein the bioactive agent comprises a peptide selected from the group consisting of fibronectin, laminin, thrombospondin, and combinations thereof.

17. The surgical implant of claim 16, wherein the surgical implant comprises an in situ forming mesh, and/or wherein the surgical implant comprises an in situ forming scaffold.

18. The surgical implant of any of claims 12 to 17, wherein the first component, the second component, or both, possess a reactive group comprising an amine; and/or wherein the first component, the second component, or both, possess a reactive group comprising a succinimide ester.

19. A method comprising:
forming a first solution comprising microparticulates having a size of from about 10 pm to about 500 pm;
forming a second solution comprising at least one cross-linking reagent;
introducing the first solution and the second solution onto tissue; and
allowing the at least one cross-linking reagent to react with the microparticulates in situ thereby forming a porous surgical implant.
